# EUROPEAN PATENT APPLICATION

(11) **EP 4 696 355 A1**
(43) Date of publication of application: **18.02.2026**
(21) Application number: 23934968.1
(22) Date of filing: 31.10.2023
(51) Int. Cl.: A61M 25/00, A61M 25/04, A61M 29/00

(54) **MEDICAL DEVICE**

(30) Priority: 23.04.2023 CN 202310445099
(71) Applicant: Shanghai MicroPort Medical (Group) Co., Ltd., Shanghai 201203 (CN)
(72) Inventor: ZHANG, Zhenghai, Shanghai 201203 (CN); ZHOU, Qi, Shanghai 201203 (CN); WU, Jintian, Shanghai 201203 (CN); CAO, Chunhong, Shanghai 201203 (CN); HU, Meng, Shanghai 201203 (CN)
(74) Representative: Marks & Clerk LLP
(86) International application number: PCT/CN2023/128109
(87) International publication number: WO 2024/221800

(57) **Abstract**

A medical device includes a sheath assembly (1000), a catheter assembly (2000) and an expandable assembly (3000). The catheter assembly (2000) is partially disposed inside the sheath assembly (1000) and includes a first tubular body (2100) axially movable relative to the sheath assembly (1000). The expandable assembly (3000) is sleeved over an outer circumferential surface of a distal end of the first tubular body (2100), and the outer surface of the expandable assembly (3000) is configured for loading a drug. The medical device is configured so that axial movement of the first tubular body (2100) relative to the sheath assembly (1000) causes the expandable assembly (3000) to be in a radially collapsed configuration in which it is confined within the sheath assembly (1000), or in a radially expanded configuration in which it is at least partially exposed outside the sheath assembly (1000). When in the radially expanded configuration, the expandable assembly (3000) comprises a first fluid passage (3001) axially extending therethrough. The medical device can treat atherosclerosis, does not block the blood flow, can prolong the treatment time, and has a good treatment effect.

## Description

### TECHNICAL FIELD

The present invention pertains to the field of apparatus for medical use. In particular, it relates to a medical device.

### BACKGROUND

Atherosclerosis, which has been regarded as one of the major contributors of heart disease and stroke, is thickening and narrowing of arteries that supply blood to various organs, including the heart, brain and kidneys. There are a number of options available for the treatment of atherosclerosis, including balloon angioplasty, stent implantation and atherectomy. Although stent implantation is mostly used, as this modality involves implanting a stent in a blood vessel for an extended period of time, it is associated with a risk of restenosis and thrombosis over time.

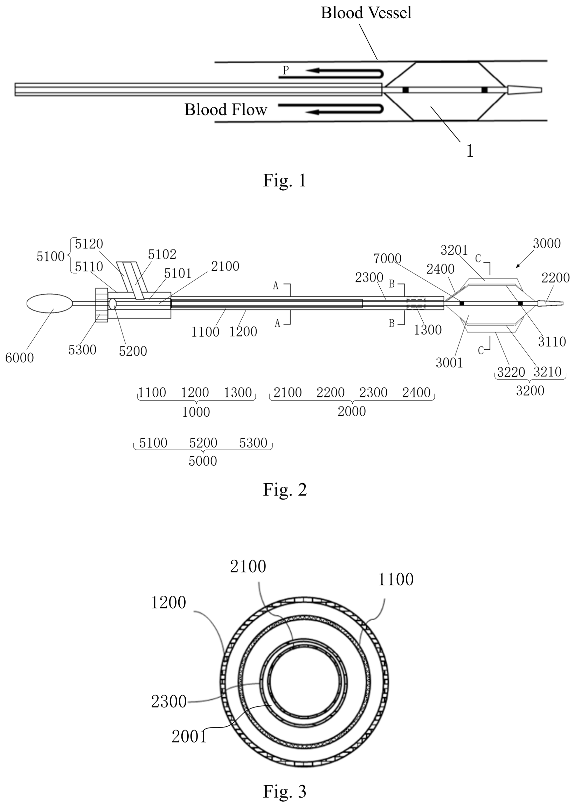

As an "implant-free" alternative, a drug-coated balloon is an interventional device using a balloon 1 as a drug carrier (see Fig. 1). The balloon 1 allows localized release of the drug to a wall of the artery, which may be able to inhibit intimal hyperplasia of the arterial wall, reducing its risk of restenosis. To date, numerous clinical trials have demonstrated the efficacy and safety of drug-coated balloons in the treatment of a wide variety of coronary, small-vessel and bifurcation stenoses. On the other hand, many experimental evidences so far have indicated that existing drug-coated balloons are associated with a number of problems, including significant drug loss, inefficient drug transfer and a short residency time in tissue at an effective concentration. These problems are primarily due to the occlusion of blood vessels and blockage of blood flow (as shown in Fig. 1, in which the arrow P indicates the direction of blood flow) when the drug-coated balloon is deployed against the vascular wall, which limits period of time allowed for therapeutic treatment.

### SUMMARY OF THE INVENTION

It is an object of the present invention to provide a medical device with improved therapeutic efficacy in the treatment of atherosclerosis and a reduced risk of restenosis and thrombosis over time.

To this end, the present invention provides a medical device comprising a sheath assembly, a catheter assembly and an expandable assembly. The catheter assembly is partially disposed inside the sheath assembly and comprises a first tubular body axially movable relative to the sheath assembly. The expandable assembly is sleeved over an outer circumferential surface of a distal end of the first tubular body, and a distal end of the expandable assembly is attached to the catheter assembly and moves synchronously with the first tubular body.

The medical device is configured so that axial movement of the first tubular body relative to the sheath assembly causes axial movement of a distal end of the expandable assembly relative to the sheath assembly, thereby bringing the expandable assembly into a radially collapsed configuration in which it is confined within the sheath assembly, or into a radially expanded configuration in which it is at least partially exposed outside the sheath assembly. The expandable assembly comprises a first fluid passage axially extending therethrough when in the radially expanded configuration.

Optionally, the expandable assembly may comprise a supporting member and a wrap element, the supporting member sleeved over the outer circumferential surface of the distal end of the first tubular body, a distal end of the supporting member attached to the catheter assembly and the supporting member moves synchronously with the first tubular body, the supporting member is a self-expanding member, the supporting member comprising a hollow structure when in a radially expanded configuration, the wrap element sleeved over a portion of an outer surface of the supporting member.

Optionally, the supporting member may comprise a supporting portion, a proximal connecting portion joined to a proximal end of the supporting portion and a distal connecting portion joined to a distal end of the supporting portion, the distal connecting portion attached to the catheter assembly, wherein the wrap element surrounds an outer surface of the supporting portion.

Optionally, the supporting portion may be a mesh tube structure, wherein the proximal connecting portion is attached to the first tubular body and comprises a plurality of proximal connecting wires arranged at intervals around a circumference of the mesh tube structure, and wherein the distal connecting portion comprises a plurality of distal connecting wires arranged at intervals around a circumference of the mesh tube structure.

Optionally, the supporting member may be fabricated by cutting a tube. Alternatively, the supporting member may be braided from wires, wherein end portions of the wires on a proximal side of the supporting portion provide the proximal connecting wires, and end portions of the wires on a distal side of the supporting portion provide the distal connecting wires.

Optionally, the sheath assembly may comprise an inner sheath and an outer sheath, the outer sheath sleeved over an outer circumferential surface of the inner sheath, the inner sheath having a proximal end flush with a proximal end of the outer sheath, the inner sheath having an axial length less than an axial length of the outer sheath,
wherein the catheter assembly is partially disposed within the inner sheath, and the expandable assembly, when in the radially collapsed configuration, is located within the outer sheath and is on a distal side of the inner sheath.

Optionally, the sheath assembly may further comprise a securing member disposed within the outer sheath and is on the distal side of the inner sheath, the securing member attached to an inner wall of the outer sheath, the securing member provided with a plurality of through bores, which extend through the securing member along an axis thereof and are arranged at intervals around a circumference of the securing member,
wherein the supporting member comprises a plurality of supporting wires arranged at intervals around an axis of the catheter assembly, each supporting wire comprising a first section, a second section and a third section, which are sequentially joined from proximal end to distal end, the first sections of the supporting wires together forming the proximal connecting portion, the first section of each supporting wire movably inserted through corresponding through bores, the third sections of the supporting wires together forming the distal connecting portion, the second sections of the supporting wires together forming the supporting portion and raised outwards along radial directions of the catheter assembly when the supporting member is in the radially expanded configuration.

Optionally, each second section may comprise a main segment and two transition segments joined to axial ends of the main segment, wherein a distance from the transition segment on a proximal side to the catheter assembly gradually increases from the proximal end to the distal end, a distance from the transition segment on a distal side to the catheter assembly gradually decreases from the proximal end to the distal end, maximum distances from the two transition segments to the catheter assembly are equal, and wherein: the main segment comprises a linear shape and a distance from the main segment to the catheter assembly is equal to the maximum distance from the transition segment to the catheter assembly; or the main segment comprises a wavy shape and a maximum distance from the main segment to the catheter assembly is greater than or equal to the maximum distance from the transition segment to the catheter assembly.

Optionally, when the supporting member is in the radially expanded configuration, the second sections may be a curved shape, wherein a distance from the second section to the catheter assembly increases and then decreases from the proximal end to the distal end.

Optionally, the catheter assembly may be provided with a second fluid passage,
wherein the wrap element comprises an inner wrap member and an outer wrap member, the inner wrap member sleeved over part of the outer surface of the supporting member, the outer wrap member sleeved over the exterior of the inner wrap member, opposite axial ends of the outer wrap member attached to axial ends of the inner wrap member, an inflation lumen formed between the outer and inner wrap members, the inflation lumen in communication with the second fluid passage.

Optionally, the second fluid passage may comprise a first fluid sub-passage and a second fluid sub-passage, which are in communication with each other,
wherein: the catheter assembly further comprises a second tubular body and a third tubular body; the first tubular body is partially inserted through the second tubular body, each of the proximal and distal ends of the first tubular body is located outside the second tubular body, the first tubular body is able to axially move relative to the second tubular body; the first fluid sub-passage is formed between an outer surface of the first tubular body and an inner surface of the second tubular body; a first seal structure is disposed between the outer surface of the first tubular body and the inner surface of the distal end of the second tubular body; a lumen of the third tubular body forms the second fluid sub-passage; a proximal end of the third tubular body is attached to the second tubular body; and a distal end of the third tubular body is inserted within the inflation lumen and is attached to the wrap element.

Optionally, the medical device may further comprise an engagement member attached to a proximal end of the sheath assembly, the engagement member comprising a housing comprising a first lumen and a second lumen, which are in communication with each other,
wherein a proximal end of the first tubular body is inserted through the first lumen and extends out of the housing, and the first tubular body is able to move along the first lumen, wherein a second seal structure is provided between the outer surface of the first tubular body and a luminal wall of a proximal end of the first lumen, wherein a proximal end of the second tubular body is attached to the luminal wall of the first lumen, and wherein the first fluid sub-passage is brought into communication with the second lumen.

Optionally, the expandable assembly may be provided with a drug coating layer.

The medical device of the present invention has the following advantages over the prior art:
It includes a sheath assembly, a catheter assembly and an expandable assembly. The catheter assembly is partially disposed inside the sheath assembly and includes a first tubular body axially movable relative to the sheath assembly. The expandable assembly is sleeved over an outer circumferential surface of a distal end of the first tubular body, and a distal end of the expandable assembly is attached to the catheter assembly and the expandable assembly moves synchronously with the first tubular body. The outer surface of the expandable assembly is configured for loading drug. The medical device is configured so that axial movement of the first tubular body relative to the sheath assembly causes axial movement of a distal end of the expandable assembly relative to the sheath assembly, thereby bringing the expandable assembly into a radially collapsed configuration in which it is confined within the sheath assembly, or into a radially expanded configuration in which it is at least partially exposed outside the sheath assembly. The expandable assembly comprises a first fluid passage axially extending therethrough when in the radially expanded configuration. During use of the medical device for treating an atherosclerosis, the first tubular body may be axially moved relative to the sheath assembly to cause the expandable assembly to expand within the blood vessel, thereby supporting a blood vessel and bringing the drug on the outer surface of the expandable assembly into contact with the blood vessel wall. In this process, the blood vessel is not occluded, and blood flow is allowed through the first fluid passage. This allows the device to prolong time for therapeutic treatment. Accordingly, the drug can be maintained in contact with the blood vessel wall for an extended period of time. Thus, the drug can be transferred more efficiently with less loss and have a longer duration in tissue at an effective concentration.

Additionally, the expandable assembly may include a supporting member and a wrap element. The supporting member may be sleeved over the outer circumferential surface of the distal end of the first tubular body, and a distal end of the supporting member may be attached to the catheter assembly and the supporting member moves synchronously with the first tubular body. The supporting member may be a self-expanding member, and may comprise a hollow structure when in a radially expanded configuration. The catheter assembly may be provided with a second fluid passage. The wrap element may include an inner wrap member and an outer wrap member. The inner wrap member may be sleeved over part of the outer surface of the supporting member, and the outer wrap member may be sleeved over the exterior of the inner wrap member. Opposite axial ends of the outer wrap member may be attached to axial ends of the inner wrap member, and an inflation lumen is formed between the outer and inner wrap members, and the inflation lumen is in communication with the second fluid passage. The drug may be loaded on the outer surface of the outer wrap member. During use of the medical device, the supporting member may be first radially expanded to cause expansion of the expandable mechanism, thereby pre-dilating the blood vessel. An inflation medium may be then introduced into the inflation lumen to inflate the wrap element, thereby additionally widening the blood vessel and bringing the outer wrap member, and hence the drug loaded thereon, into closer contact with a wall of the blood vessel. In this way, damage to the blood vessel wall possibly caused by instantaneous expansion force during a single dilation can be effectively reduced, making the treatment safer.

### BRIEF DESCRIPTION OF THE DRAWINGS

The accompanying drawings are provided to facilitate a better understanding of the present invention and do not unduly limit the scope thereof, in which:
Fig. 1 schematically illustrates a conventional drug-coated balloon occludes a blood vessel and blocks blood flow therein during its use;
Fig. 2 is a schematic diagram of a medical device according to an embodiment of the present invention, showing an expandable assembly, which is at least partially exposed outside a sheath assembly and therefore comprises a radially expanded configuration, and a supporting member including multiple supporting wires;
Fig. 3 is a cross-sectional view of the medical device taken along A-A of Fig. 2;
Fig. 4 is a cross-sectional view of the medical device taken along B-B of Fig. 2;
Fig. 5 is a cross-sectional view of the medical device taken along C-C of Fig. 2;
Fig. 6 is a schematic diagram of a medical device according to an embodiment of the present invention, showing an expandable assembly, which is confined within a sheath assembly and therefore comprises a radially collapsed configuration;
Fig. 7 is a schematic diagram of a medical device according to an embodiment of the present invention, showing an expandable assembly, which is exposed outside a sheath assembly and therefore comprises a radially expanded configuration, a drug coating layer and a supporting member including multiple supporting wires;
Fig. 8 is a cross-sectional view of the medical device taken along D-D of Fig. 7;
Fig. 9 is a schematic diagram of a medical device according to an embodiment of the present invention, showing an expandable assembly, which is exposed outside a sheath assembly and therefore comprises a radially expanded configuration, and a supporting member including a supporting portion in the form of a mesh tube structure, but not showing a drug coating layer;
Fig. 10 schematically illustrates an application scenario of a medical device according to an embodiment of the present invention;
Fig. 11 is a schematic diagram of a sheath assembly and an engagement member in a medical device according to an embodiment of the present invention;
Fig. 12 is a cross-sectional view of the medical device taken along E-E of Fig. 11;
Fig. 13 is a schematic partial view of a medical device according to an embodiment of the present invention, showing a supporting member including supporting wires;
Fig. 14 is a schematic diagram of a supporting wire of a supporting member in a medical device according to an embodiment of the present invention, which has a second section including a straight main segment;
Fig. 15 is a schematic diagram of a supporting wire of a supporting member in a medical device according to an embodiment of the present invention, which has a second section including a wavy main segment;
Fig. 16 is a schematic diagram of a supporting wire of a supporting member in a medical device according to an embodiment of the present invention, which has a curved second section;
Fig. 17 is a schematic diagram of a sheath assembly and an engagement member in a medical device according to an embodiment of the present invention, in which the sheath assembly differs from that of Fig. 11 in not including a securing member;
Fig. 18 is a schematic partial view of a medical device according to an embodiment of the present invention, showing a supporting member fabricated by cutting a tube;
Fig. 19 is a schematic flattened view of a supporting member in a medical device according to an embodiment of the present invention, which is fabricated by cutting a tube;
Fig. 20 is a schematic partial view of a medical device according to an embodiment of the present invention, showing a supporting member braided from wires; and
Fig. 21 is a schematic diagram of a supporting member in a medical device according to an embodiment of the present invention, which is braided from wires.

### List of Reference Numerals

1, balloon; 1000, sheath assembly; 1100, inner sheath; 1200, outer sheath; 1300, securing member; 1310, through bore; 2000, catheter assembly; 2001, first fluid sub-passage; 2100, first tubular body; 2200, guide head; 2300, second tubular body; 2400, third tubular body; 3000, expandable assembly; 3001, first fluid passage; 3100, supporting member; 3110, supporting wire; 3111, first section; 3112, second section; 3112a, proximal transition segment; 3112b, main segment; 3112c, distal transition segment; 3113, third section; 3120, mesh tube structure; 3130, proximal connecting wire; 3140, distal connecting wire; 3200, wrap element; 3210, inner wrap member; 3220, outer wrap member; 3201, inflation lumen; 4000, drug coating layer; 5000, engagement member; 5100, housing; 5101, first lumen; 5102, second lumen; 5110, first branch; 5120, second branch; 5200, sealing ring; 5300, tightening knob; 6000, force-bearing portion; 7000, radiopaque element.

### DETAILED DESCRIPTION

Embodiments of the present invention will be described below by way of specific examples. Based on the disclosure and teachings provided herein, a person of ordinary skill in the art will realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

It is an object of the present invention to provide a medical device, which can be used to treat atherosclerosis. In use, the medical device is able to widen a blood vessel and bring a drug thereon into contact with the wall of the blood vessel, while allowing blood to flow therethrough. This makes it unnecessary to block blood flow, allowing the medical device to be deployed for a longer time to deliver therapeutic treatment. As a result, less drug loss, more efficient drug transfer and an extension of the duration of effective drug concentration in tissues can be achieved.

As used herein, the terms "proximal end" and "distal end" may be used to describe components or portions of the medical device relative to an operator during its normal use. While not intended to be limiting, a "distal end" usually refers to an end that enters the body of a patient first, while a "proximal end" usually refers to an end closer to the operator, during use of the medical device.

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. Throughout these drawings, same numerals indicate same elements.

Figs. 2, 6, 7 and 9 are schematic diagrams showing the structure of a medical device according to embodiments of the present invention. As shown in Figs. 2, 6, 7 and 9, the medical device includes a sheath assembly 1000, a catheter assembly 2000 and an expandable assembly 3000. The catheter assembly 2000 is partially disposed inside the sheath assembly 1000 and includes a first tubular body 2100 that is axially movable relative to the sheath assembly 1000. The expandable assembly 3000 is disposed over an outer circumferential surface of the first tubular body 2100 around a distal end thereof. The expandable assembly 3000 comprises a distal end attached to the catheter assembly 2000 and moves synchronously with the first tubular body 2100. An outer surface of the expandable assembly 3000 is configured for loading of a drug. The medical device is configured so that axial movement of the first tubular body 2100 relative to the sheath assembly 1000 causes axial movement of the distal end of the expandable assembly 3000 relative to the sheath assembly 1000, which in turn causes the expandable assembly 3000 to be confined within the sheath assembly 1000 or at least partially exposed outside the sheath assembly 1000. When confined within the sheath assembly 1000, the expandable assembly 3000 is in a radially collapsed configuration. When at least partially exposed outside the sheath assembly 1000, the expandable assembly 3000 is in a radially expanded configuration. When in the radially expanded configuration, the expandable assembly 3000 comprises a first fluid passage 3001 axially extending therethrough.

By "the expandable assembly 3000 comprises a first fluid passage 3001 axially extending therethrough", it is intended to mean that the first fluid passage 3001 communicates with the outside world at both axial ends of the medical device, allowing a fluid to flow into the first fluid passage 3001 through one end of the expandable assembly 3000 and out of the expandable assembly 3000 from the other end of the first fluid passage 3001. In this sense, the presence of obstructions that reduce flux areas at both axial ends of the first fluid passage 3001 is permitted. In other words, any configuration is possible as long as it ensures that a fluid can flow from the outside world into the first fluid passage 3001 through one end thereof and out of the first fluid passage 3001 from the other end.

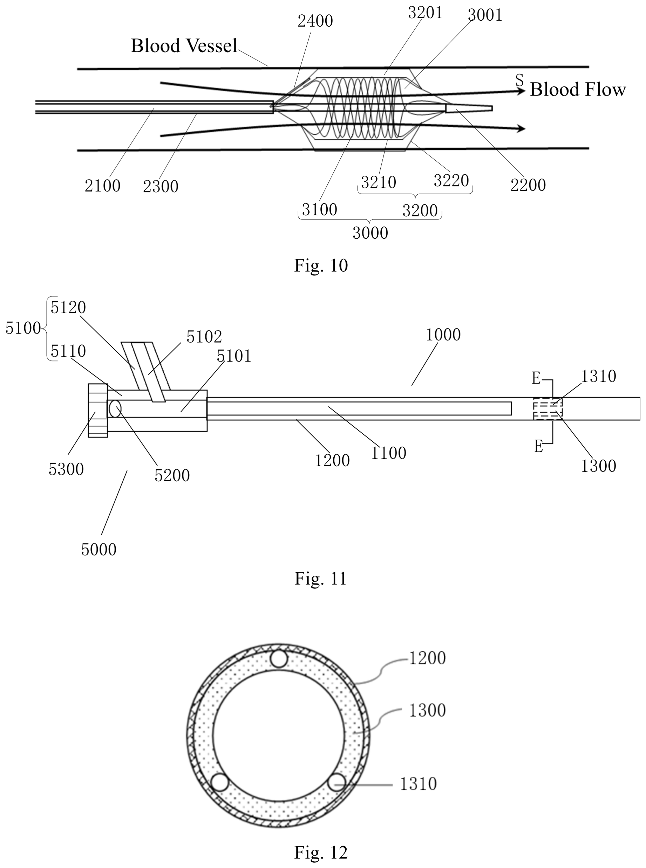

Accordingly, during use of the medical device for treatment of atherosclerosis, first of all, with the expandable assembly 3000 being confined within the sheath assembly 1000, the medical device is advanced until its distal end reaches a lesion site within a blood vessel. Axial movement of the sheath assembly 1000 and the first tubular body 2100 relative to each other is then caused, leading to the expandable assembly 3000 being at least partially exposed outside the sheath assembly 1000. In this configuration, the expandable assembly 3000 can be switched to inflated configuration and the diseased blood vessel can be widened by the expandable assembly 3000 with an inflated configuration. In the case of a drug being loaded on its outer surface, to allow the drug to act on the wall of the blood vessel. The drug may have an effect of inhibiting hyperplasia of the blood vessel. After the expandable assembly 3000 is inflated, blood can flow through the first fluid passage 3001, as indicated by the arrow S in Fig. 10. Thus, unlike conventional drug-coated balloons, the medical device of the present invention can widen a blood vessel while not blocking blood flow therein. This allows the medical device to be deployed for a longer time to deliver therapeutic treatment and hence enables a drug loaded on the expandable assembly 3000 to be more efficiently transferred with less loss, resulting in a longer residency time thereof in tissue at an effective concentration.

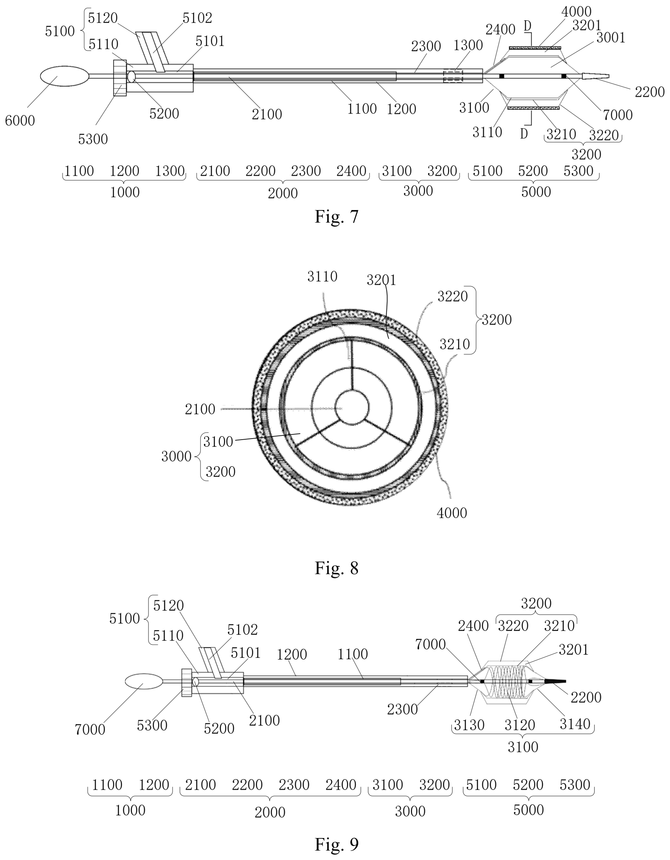

Preferably, as shown in Figs. 7 and 8, on the outer surface of the expandable assembly 3000 may be loaded a drug in the form of an active ingredient, or a pharmaceutical composition. Examples of the active ingredient may include at least one of rapamycin, paclitaxel, everolimus, deforolimus, temsirolimus and zotarolimus. The pharmaceutical composition may contain the active ingredient and a sustained-release polymer including at least one of polylactic acid, poly(glycolic acid), gelatin, chitosan, poly(lactic acid-co-glycolic acid) and polyethylene glycol.

In practice, the drug may be prepared as a solution and then applied to the outer surface of the expandable assembly 3000 by ultrasonic spraying, dip coating, crystal growth or any other suitable approach. After the solution dries, a drug coating 4000 (see Fig. 8) will be present on the outer surface of the expandable assembly 3000, thus achieving loading of the drug on the expandable assembly 3000.

Structure details of the medical device will be set forth below.

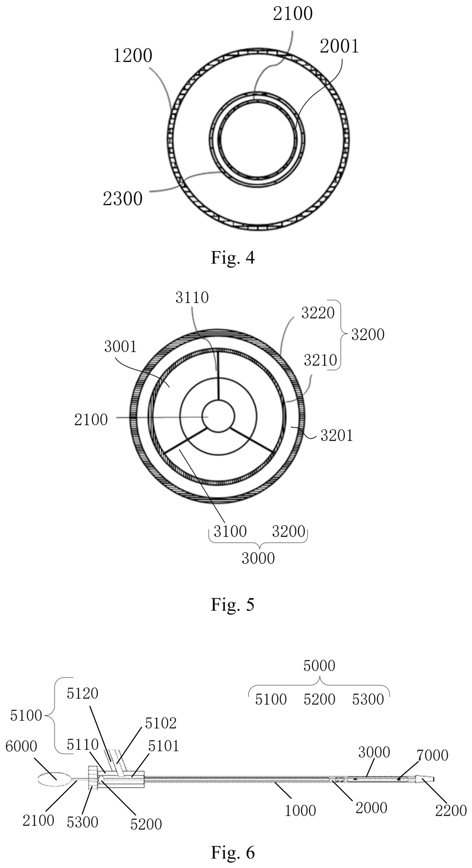

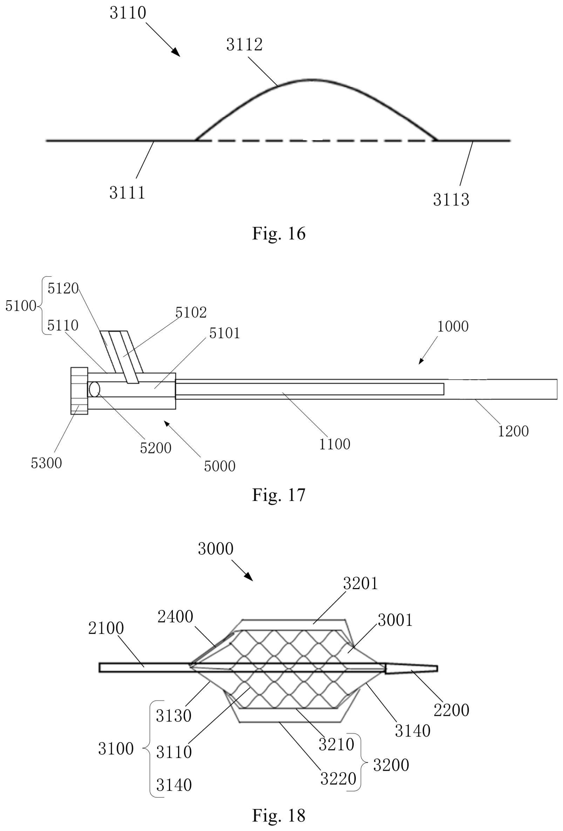

Generally, a proximal section of the catheter assembly 2000 has an outer diameter less than an outer diameter of the expandable assembly 3000. Referring to Figs. 11 and 17, in a preferred embodiment, the sheath assembly 1000 includes an inner sheath 1100 and an outer sheath 1200 disposed over an outer circumferential surface of the inner sheath 1100. A proximal end of the outer sheath 1200 is flush with a proximal end of the inner sheath 1100, and an axial length of the inner sheath 1100 is less than an axial length of the outer sheath 1200. Accordingly, a distal end of the inner sheath 1100 is within the outer sheath 1200. As shown in Figs. 2, 6, 7 and 9, part of the catheter assembly 2000 is disposed in a lumen of the inner sheath 1100. As shown in Fig. 6, when confined within the sheath assembly 1000, the expandable assembly 3000 is located within a lumen of the outer sheath 1200 and is on a distal side of the inner sheath 1100. With this arrangement, a small clearance is left between the proximal section of the catheter assembly 2000 and the sheath assembly 1000, and the catheter assembly 2000 is radially restricted by the inner sheath 1100. This can reduce bending of the catheter assembly 2000 within the sheath assembly 1000 and facilitate relative axial movement of the expandable mechanism and the sheath assembly 1000.

In this embodiment, the inner sheath 1100 is a multi-layer tube structure. In one optional implementation, the inner sheath 1100 includes an inner liner, an intermediate layer and an outer jacket (not shown), which are sequentially arranged in a radial direction. The intermediate layer is a braided layer to improve flexibility of the inner sheath 1100. Each of the inner liner and the outer jacket may be made of any suitable polymer material. The outer sheath 1200 may be made of at least one of polyamide, polyether block amide and polyurethane.

The catheter assembly 2000 further includes a guide head 2200 attached to a distal end of the first tubular body 2100. Generally, it is a tapered structure with its outer diameter gradually decreasing from proximal end to distal end, and is provided to guide the medical device into a blood vessel. The guide head 2200 always remains external to a distal end of the sheath assembly 1000. Preferably, the guide head 2200 is made of a polymer material, such as polyether block amide, high-density polyethylene or polyamide.

Referring to Figs. 2, 7 and 9, the expandable assembly 3000 includes a supporting member 3100 and a wrap element 3200. The supporting member 3100 is disposed over the outer circumferential surface of the catheter assembly 2000 around the distal end thereof, and the wrap element 3200 covers at least part of an outer surface of the supporting member 3100. A distal end of the supporting member 3100 is the distal end of the expandable assembly 3000, and may be attached to the guide head 2200 by laser welding or any other suitable approach so as to remain stationary relative to the guide head 2200 and the first tubular body 2100. With this arrangement, the distal end of the supporting member 3100 is movable in synchronization with the first tubular body 2100, and enable the distal end of the expandable assembly 3000 to be movable in synchronization with the first tubular body 2100.

According to embodiments of the present invention, the supporting member 3100 is a self-expanding structure. By "self-expanding member", it is intended to mean that the supporting member 3100 is made of a highly elastic material and pre-shaped into an expanded configuration. When subjected to a radial compressive force, the supporting member 3100 may radially contract while storing elastic potential energy. Upon the radial compressive force being removed, the supporting member 3100 will release the stored elastic potential energy and recover the expanded configuration.

With this arrangement, when the supporting member 3100 is not subjected to any external radial compressive force, it remains in a radially expanded configuration and maintains the expandable assembly 3000 in the radially expanded configuration. However, when subjected to an external radial compressive force, the supporting member 3100 may be radially collapsed, putting the expandable assembly 3000 into the radially collapsed configuration. The supporting member 3100 may be subjected to a radial compressive force when the expandable assembly 3000 is confined within the sheath assembly 1000. Once the expandable assembly 3000 is at least partially exposed outside the sheath assembly 1000, at least part of the supporting member 3100 (e.g., the supporting portion, as described below) will no longer be subjected to the radial compressive force from the sheath assembly 1000.

Examples of the highly elastic material may include, but are not limited to, shape memory alloys. More specific examples may include gold-cadmium alloys, copper-zinc alloys, aluminum-nickel alloys, platinum-iron alloys and nickel-titanium alloys, etc. It is preferred to be a nickel-titanium alloy. When radially expanded, the supporting member 3100 may comprise a hollow structure and the expandable assembly 3000 comprises the first fluid passage 3001 when part of the outer surface of the supporting member 3100 is covered by the wrap element 3200. The wrap element 3200 may be attached to the supporting member 3100 by laser welding or any other suitable approach. To this end, the highly elastic material, from which the supporting member 3100 is fabricated, may be provided with a polymer coating layer made of the same or a similar material as the wrap 3200.

The supporting member 3100 includes a supporting portion, a proximal connecting portion joined to a proximal end of the supporting portion and a distal connecting portion joined to a distal end of the supporting portion, along an axis of the medical device. The distal connecting portion is attached to the guide head 2200. The wrap element 3200 is disposed over at least part of an outer surface of the supporting portion.

In an optional embodiment, referring to Figs. 2, 7, 11 and 12, the sheath assembly 1000 further includes a securing member 1300 provided inside the outer sheath 1200 and is on the distal side of the inner sheath 1100. The securing member 1300 is attached to an inner wall of the outer sheath 1200 and comprises multiple through bores 1310 axially extending therethrough, which are arranged at intervals around circumference of the securing member 1300.

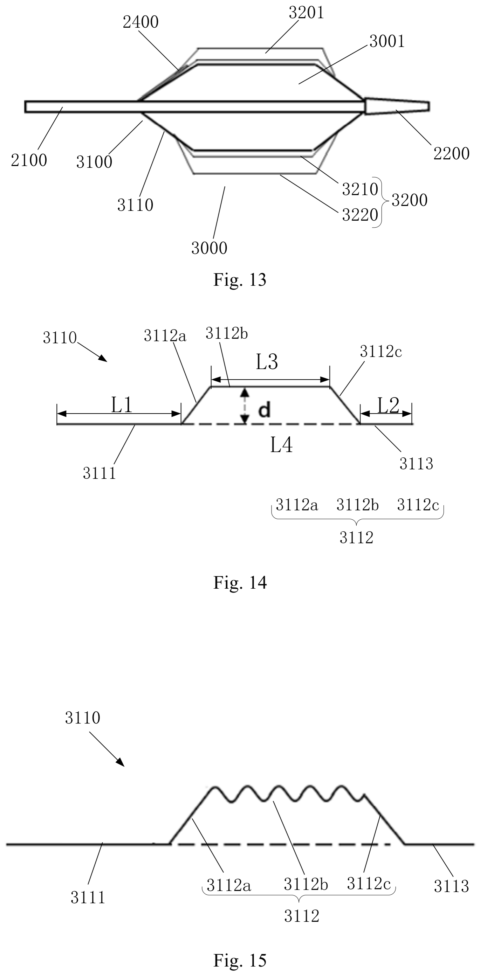

The supporting member 3100 includes multiple supporting wires 3110 (e.g., 3-6 supporting wires), each of which is preferred to have a diameter of 0.1-0.5 mm, such as 0.3 mm. The multiple supporting wires 3110 are provided at the distal end of the first tubular body 2100 and arranged at intervals around an axis of the first tubular body 2100 so that the supporting member 3100 is disposed over the outer circumferential surface of the first tubular body 2100 around the distal end thereof. As shown in Figs. 14 to 16, each supporting wire 3110 includes a first section 3111, a second section 3112 and a third section 3113, which are sequentially joined from proximal end to distal end. The first sections 3111 of the supporting wires 3110 together form the proximal connecting portion and are movably inserted through the respective through bores 1310. The third sections 3113 of the supporting wires 3110 together form the distal connecting portion, and the second sections 3112 of the supporting wires 3110 together form the supporting portion. The first sections 3111 form obstructions, which reduce a flux area at a proximal end of the first fluid passage 3001. Likewise, the third sections 3113 form obstructions, which reduce a flux area at a distal end of the first fluid passage 3001. In this embodiment, since the first sections 3111 are needed to be inserted through the through bores 1310, they usually have a length L1 larger than a length L2 of the third sections 3113. The length L1 of the first sections 3111 typically ranges from 1 mm to 50 mm, and the length L2 of the third sections 3113 may range from 1 mm to 10 mm.

When the supporting member 3100 is in the radially expanded configuration, the second sections 3112 are raised outwards along radial directions of the catheter assembly 2000. By "the second sections 3112 are raised outwards along radial directions of the catheter assembly 2000", it is intended to mean that the maximum distance from the second section 3112 to the first tubular body 2100 is greater than the distance from the first section 3111 to the first tubular body 2100 and is greater than the distance from the third section 3113 to the first tubular body 2100.

In one optional implementation, as shown in Fig. 14, the second section 3112 includes a proximal transition segment 3112a, a main segment 3112b and a distal transition segment 3112c, which are sequentially joined from proximal end to distal end. When the supporting member 3100 is in the radially expanded configuration, a distance from the proximal transition segment 3112a to the catheter assembly 2000 gradually increases from proximal end to distal end, while a distance from the distal transition segment 3112c to the catheter assembly 2000 gradually decreases from proximal end to distal end. The maximum distance from the distal transition segment 3112c to the catheter assembly 2000 is same as the maximum distance from the proximal transition segment 3112c to the catheter assembly 2000. The main segment 3112b is in the shape of a straight line and a distance from the main segment 3112b to the catheter assembly 2000 is equal to the maximum distance from the proximal transition segment 3112a to the catheter assembly 2000. In this embodiment, a dimension L3 of the main segment 3112b along the axis of the medical device may be 5 mm to 50 mm, preferably 6 mm to 48 mm. The distance d from the main segment 3112b to the catheter assembly 2000 may depend on the size of a diseased blood vessel. In particular, it may range from 0.5 mm to 100 mm, such as 1.75 mm. A distance L4 from a proximal end of the proximal transition segment 3112a to a distal end of the distal transition segment 3112c may be 4 mm greater than the dimension L3 of the main segment 3112b. Alternatively, as shown in Fig. 15, the main segment 3112b may have a wavy shape, such as a sinusoidally wavy shape. The distance from the main segment 3112b to the catheter assembly 2000 may be greater than or equal to the maximum distance from the proximal transition segment 3112a to the catheter assembly 2000.

In an alternative implementation, as shown in Fig. 16, when the supporting member 3100 is in the radially expanded configuration, the second section 3112 may be curved and a distance from the second section 3112 to the catheter assembly 2000 increases and then decreases from proximal end to distal end.

It would be appreciated that when the supporting member 3100 is in the radially collapsed configuration, under the action of the radial compressive force, the supporting wires 3110 are stretched and lengthened into a straight or almost straight linear shape. Therefore, the axial length of the supporting member 3100 varies when it is switched between the radially expanded and collapsed configurations. Thus, when the supporting member 3100 switches between the radially expanded and collapsed configurations, the proximal ends of the supporting wires 3110 moves along an axis of the catheter assembly 2000. According to the present invention, by configuring the securing member 1300 and inserting the first sections 3111 of the supporting wires 3110 through the respective through bores 1310, the through bores 1310 can guide movement of the proximal ends of the supporting wires 3110. In addition, multiple through bores 1310 isolate multiple supporting wires 3110 so that entanglement of the multiple supporting wires 3110 can be avoided.

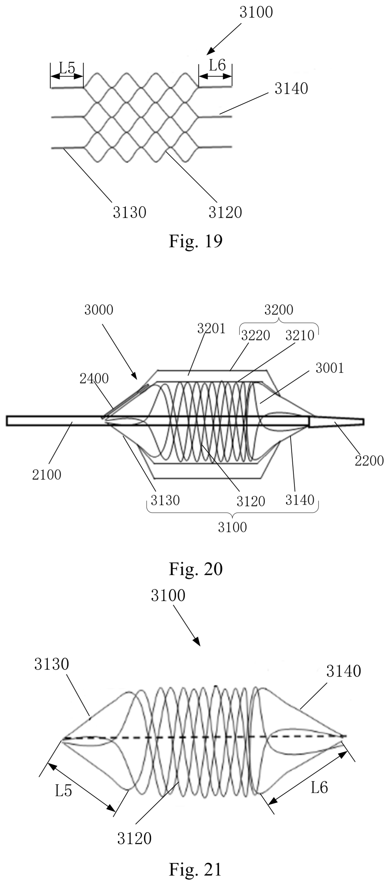

In another alternative embodiment, as shown in Figs. 9, 10 and 18 to 21, the supporting portion comprises a mesh tube structure 3120, which can provide continuous support to the wrap element 3200 over the entire circumference, enabling desirable contact of the wrap element 3200 with a blood vessel wall. In this embodiment, as shown in Fig. 17, the securing member may be omitted from the sheath assembly 1000, and the proximal connecting portion may be directly connected to the first tubular body 2100. In addition, the proximal connecting portion includes multiple connecting wires 3130 spaced circumferentially around the mesh tube structure 3120, and the distal connecting portion includes multiple distal connecting wires 3140 spaced circumferentially around the mesh tube structure 3120. In this case, a lumen of the mesh tube structure 3120 forms the first fluid passage 3001. Although the proximal connecting wires 3130 form obstructions that reduce a flux area at the proximal end of the first fluid passage, a fluid still can enter the first fluid passage from the proximal end thereof. Similarly, although the distal connecting wires 3140 form obstructions that reduce a flux area at the distal end of the first fluid passage, the fluid still can flow out of the first fluid passage from its distal end.

Typically, the number of proximal connecting wires 3130 is two to six, such as three. Preferably, the number of distal connecting wires 3140 is equal to the number of proximal connecting wires 3130, and the locations of the distal connecting wires 3140 are symmetrical to those of the respective proximal connecting wires 3130. That is to say, each of the distal connecting wires 3140 is aligned with a respective one of the proximal connecting wires 3130. A radius of the mesh tube structure 3120 may depend on the size of a diseased blood vessel. In particular, it may range from 0.1 mm to 100 mm. A length L5 of the proximal connecting wires 3130 typically ranges from 1 mm to 50 mm, and a length L6 of the distal connecting wires 3140 may range from 1 mm to 10 mm. The length of the proximal connecting wires 3130 may be greater than a length of the distal connecting wires 3140.

Each of the supporting portion, the proximal connecting portion and the distal connecting portion of the supporting member 3100 may be fabricated from a tube using laser cutting or any other suitable cutting technique. Preferably, the resulting mesh tube structure 3120 has openings surrounded by supporting wires in the shape of portions of sinusoidal waves. This can impart good flexibility to the supporting member 3100 and better adaptability to various complex vascular lesions.

Alternatively, the supporting member 3100 may be braided from one to six wires, preferably four wires. In this case, the wires may be braided in such a manner that each of them has two free end portions on opposite sides of the resulting mesh tube structure 3120. The wire portions on a proximal side of the mesh tube structure 3120 form the proximal connecting wires 3130, and wire portions on a distal side of the mesh tube structure 3120 form the distal connecting wires 3140. It will be understood that the braided mesh tube structure 3120 will not come apart after the proximal connecting wires 3130 and the distal connecting wires 3140 are attached to the first tubular body 2100, respectively.

In an improved embodiment, as shown in Figs. 2, 5, 7 to 10, 13, 18 and 20, the wrap element 3200 comprises an inflation lumen 3201, and an inflation medium can be introduced into the inflation lumen 3201 to inflate the wrap element 3200. The wrap element 3200 will radially contract as the inflation medium is discharged from the inflation lumen 3201. The drug coating layer 4000 may be provided on an outer surface of an outer wrap member 3220 in the wrap element 3200 to load the drug. With this arrangement, during use of the medical device for treating a diseased blood vessel, the first tubular body 2100 is first axially moved relative to the sheath assembly 1000 to expand the expandable assembly 3000 and hence widen the blood vessel to some extent, achieving pre-dilation of the blood vessel. The inflation medium is then introduced into the inflation lumen 3201 of the wrap element 3200, further stretching the outer wrap member 3220 and additionally widening blood vessel. At the same time, a contact force between the drug coating layer 4000 on the outer surface of the outer wrap member 3220 and the wall of the blood vessel can be increased. By doing this, not only damage to the blood vessel wall possibly caused by spontaneous expansion force during the radial expansion of the expandable assembly can be reduced, better contact of the drug with the blood vessel wall can be achieved, resulting in improved efficacy.

In particular, the wrap element 3200 may include an inner wrap member 3210 and an outer wrap member 3220. The inner wrap member 3210 is disposed over part of the outer surface of the supporting member 3100, for example, at least over the outer surface of the supporting portion. The outer wrap member 3220 is disposed over an outer surface of the inner wrap member 3210, and opposite axial ends of the outer wrap member 3220 are attached to axial ends of the inner wrap member 3210. Further, the inflation lumen 3201 may be formed between the outer wrap member 3220 and the inner wrap member 3210.

It will be understood that the inner wrap member 3210 is stretched when the supporting member 3100 is in the radially expanded configuration, and the outer wrap member 3220 is stretched when the wrap 3200 is inflated. When the inner wrap member 3210 and the outer wrap member 3220 are both stretched, the outer wrap member 3220 may have an inner diameter approximately 0.5 mm greater than an outer diameter of the inner wrap member 3210. For example, in one particular implementation, the outer diameter of the stretched inner wrap member 3210 is 3.5 mm, and the inner diameter of the stretched outer wrap member 3220 is 4 mm. In the present embodiment, a maximum distance from the supporting portion of the supporting member 3100 to the catheter assembly 2000 is half of the inner diameter of the inner wrap member 3210.

The inner wrap member 3210 and the outer wrap member 3220 may be made of the same or different materials. Particular examples of the materials may include, but are not limited to, polyamide, polyether block amide and polyurethane. The inner wrap member 3210 and the outer wrap member 3220 may be integrally formed, or separately fabricated and then attached to each other by bonding or any other suitable approach.

The catheter assembly 2000 comprises a second fluid passage (not labeled) in structural correspondence with the wrap element 3200. The second fluid passage is brought into communication with the inflation lumen 3201 and configured for passage of the inflation medium therethrough, allowing the inflation medium to be introduced into and discharged from the inflation lumen 3201.

Optionally, the second fluid passage includes a first fluid sub-passage 2001 (as shown in Figs. 3 and 4) and a second fluid sub-passage (not shown), which are communication with each other. In one exemplary embodiment, as shown in Figs. 2, 3, 4, 7 and 9, the catheter assembly 2000 further includes a second tubular body 2300 and a third tubular body 2400. The first tubular body 2100 is inserted through the second tubular body 2300 with its proximal and distal ends being both exposed out of the second tubular body 2300, and is axially movable relative to the second tubular body 2300. The first fluid sub-passage 2001 is formed between the outer surface of the first tubular body 2100 and an inner surface of the second tubular body 2300. The third tubular body 2400 comprises a lumen, which forms the second fluid sub-passage. A proximal end of the third tubular body 2400 is attached to the second tubular body 2300 by laser welding, bonding or any other suitable approach, and a distal end of the third tubular body 2400 is inserted within the inflation lumen 3201 and attached to the wrap element 3200.

It will be understood that a first seal structure (not shown) may be provided between the outer surface of the first tubular body 2100 and the inner surface of the second tubular body 2300 around a distal end of the second tubular body 2300 to prevent leakage of the inflation medium, and the first seal structure does not interfere with the relative axial movement of the first tubular body 2100 and the second tubular body 2300. The first tubular body 2100 and the second tubular body 2300 may be made of the same material, examples of which may include, but are not limited to, polyamide, polyether block amide and polytetrafluoroethylene. Examples of a material, from which the third tubular body 2400 can be fabricated, may include, but are not limited to, any of polyamide, polyether block amide and polyurethane.

Referring again to Figs. 2 to 4, 7 and 9, at least part of the second tubular body 2300 is disposed inside the inner sheath 1100. Therefore, the inner sheath 1100 is provided to radially restrict the second tubular body 2300 to reduce its bending within the sheath assembly 1000, facilitating axial movement of the first tubular body 2100 relative to the second tubular body 2300.

In addition, as shown in Figs. 2, 6, 7, 9, 11 and 17, the medical device further includes an engagement member 5000, which is disposed at a proximal end of the sheath assembly 1000 and includes a housing 5100 connected to both the proximal end of the inner sheath 1100 and the proximal end of the outer sheath 1200. The housing 5100 includes a first branch 5110 and a second branch 5120. The housing 5100 comprises a first lumen 5101 and a second lumen 5102, which are in communication with each other. The first lumen 5101 axially extends through the first branch 5110 and communicates with the inner sheath 1100. The second lumen 5102 axially extends through the second branch 5120 and communicates with an external inflation medium mechanism, which may be, for example, a pump, syringe or the like. A proximal end of the first tubular body 2100 is inserted through the first lumen 5101 and extends to exterior of a proximal end of the housing 5100, and the first tubular body 2100 can be movable along the first lumen 5101. A proximal end of the second tubular body 2300 may be positioned within the first lumen 5101 and attached to a luminal wall of the first lumen 5101 by photocuring or any other suitable approach. The second fluid passage of the catheter assembly 2000 is brought into communication with the second lumen 5102 so that the inflation medium can flow through the second fluid passage and the second lumen 5102.

In order to prevent leakage of the inflation medium from the first lumen 5101, a second seal structure, such as a sealing ring 5200, may be provided between the outer surface of the first tubular body 2100 and the luminal wall of the first lumen 5101 around a proximal end of the first lumen 5101. It will be understood that the presence of the second seal structure does not hinder either of the fluid communication between the second fluid passage and the second lumen 5102 and the movement of the first tubular body 2100. The engagement member 5000 further includes a tightening knob 5300 arranged at and threadedly coupled to a proximal end of the first branch 5110. Interaction of the sealing ring 5200 with the luminal wall of the first lumen 5101 and the first tubular body 2100 can be enhanced by turning the tightening knob 5300, thereby providing an improved sealing effect.

With continued reference to Figs. 2, 6, 7, 9, 11 and 17, the medical device further includes a force-bearing portion 6000 coupled to the proximal end of the first tubular body 2100. The force-bearing portion 6000 is configured for an external force to be applied thereto to drive the first tubular body 2100 to axially move relative to the sheath assembly 1000 to switch the expandable assembly 3000 between the radially expanded and collapsed configurations. Specifically, fabrication of the medical device may involve forming the drug coating layer 4000 on the outer surface of the wrap element 3200 and then folding and crimping the wrap element 3200. The force-bearing portion 6000 is then retracted to enable the first tubular body 2100 to move in a direction from the distal end to the proximal end relative to the sheath assembly 1000 until the expandable assembly 3000 is confined within the outer sheath 1200. During use of the medical device, after the expandable assembly 3000 is advanced to a target lesion site in an intended blood vessel, the force-bearing portion 6000 is advanced to enable the first tubular body 2100 to move in a direction from the distal end to the proximal end relative to the sheath assembly 1000. As a result, the expandable assembly 3000 is at least partially exposed outside the sheath assembly 1000 and radially expands. The force-bearing portion 6000 may have any suitable shape that allows it to be conveniently held by an operator, such as a spherical, ellipsoidal or a circular disk-like shape.

As shown in Figs. 2, 6, 7 and 9, the medical device further includes a radiopaque element 7000 configured to show the position of the expandable assembly 3000. The radiopaque element 7000 may be provided on the first tubular body 2100 in positional correspondence with the expandable assembly 3000.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical device, comprising a sheath assembly, a catheter assembly and an expandable assembly, wherein the catheter assembly is partially disposed inside the sheath assembly and comprises a first tubular body axially movable relative to the sheath assembly, and wherein the expandable assembly is sleeved over an outer circumferential surface of a distal end of the first tubular body, a distal end of the expandable assembly is attached to the catheter assembly and moves synchronously with the first tubular body,
wherein the medical device is configured so that an axial movement of the first tubular body relative to the sheath assembly causes an axial movement of a distal end of the expandable assembly relative to the sheath assembly, thereby bringing the expandable assembly into a radially collapsed configuration in which the expandable assembly is confined within the sheath assembly, or into a radially expanded configuration in which the expandable assembly is at least partially exposed outside the sheath assembly, wherein the expandable assembly comprises a first fluid passage axially extending therethrough when in the radially expanded configuration.

2. The medical device according to claim 1, wherein the expandable assembly comprises a supporting member and a wrap element, wherein the supporting member is sleeved over the outer circumferential surface of the distal end of the first tubular body, wherein a distal end of the supporting member is attached to the catheter assembly and the supporting member moves synchronously with the first tubular body, wherein the supporting member is a self-expanding member, wherein the supporting member comprises a hollow structure when in a radially expanded configuration, and wherein the wrap element is sleeved over a portion of an outer surface of the supporting member.

3. The medical device according to claim 2, wherein the supporting member comprises a supporting portion, a proximal connecting portion joined to a proximal end of the supporting portion and a distal connecting portion joined to a distal end of the supporting portion, wherein the distal connecting portion is attached to the catheter assembly, and wherein the wrap element surrounds an outer surface of the supporting portion.

4. The medical device according to claim 3, wherein the supporting portion is a mesh tube structure, wherein the proximal connecting portion is attached to the first tubular body and comprises a plurality of proximal connecting wires arranged at intervals around a circumference of the mesh tube structure, and wherein the distal connecting portion comprises a plurality of distal connecting wires arranged at intervals around a circumference of the mesh tube structure.

5. The medical device according to claim 4, wherein the supporting member is fabricated by cutting a tube, or wherein the supporting member is braided from wires, wherein a portion of the wire on a proximal side of the supporting portion forms the proximal connecting wire, and wherein a portion of the wire on a distal side of the supporting portion forms the distal connecting wire.

6. The medical device according to claim 3, wherein the sheath assembly comprises an inner sheath and an outer sheath, wherein the outer sheath is sleeved over an outer circumferential surface of the inner sheath, wherein the inner sheath having a proximal end flush with a proximal end of the outer sheath, and wherein the inner sheath having an axial length less than an axial length of the outer sheath,
wherein the catheter assembly is partially disposed within the inner sheath, and wherein the expandable assembly is located within the outer sheath and is on a distal side of the inner sheath when in the radially collapsed configuration.

7. The medical device according to claim 6, wherein the sheath assembly further comprises a securing member disposed within the outer sheath and is on the distal side of the inner sheath, wherein the securing member is attached to an inner wall of the outer sheath, wherein the securing member is provided with a plurality of through bores which extend through the securing member along an axis thereof, and wherein the plurality of through bores are arranged at intervals around a circumference of the securing member,
wherein the supporting member comprises a plurality of supporting wires arranged at intervals around an axis of the catheter assembly, wherein each supporting wire comprises a first section, a second section and a third section, which are sequentially joined from a proximal end to a distal end, wherein the first sections of the plurality of supporting wires form the proximal connecting portion, wherein the first section of each supporting wire is movably inserted through a corresponding through bore, wherein the third sections of the plurality of supporting wires form the distal connecting portion, and wherein the second sections of the plurality of supporting wires form the supporting portion and are raised outwards along a radial direction of the catheter assembly when the supporting member is in the radially expanded configuration.

8. The medical device according to claim 7, wherein the second section comprises a main segment and two transition segments joined to axial ends of the main segment, wherein a distance from the transition segment on a proximal side to the catheter assembly gradually increases from the proximal end to the distal end, a distance from the transition segment on a distal side to the catheter assembly gradually decreases from the proximal end to the distal end, maximum distances from the two transition segments to the catheter assembly are equal, and wherein: the main segment comprises a linear shape and a distance from the main segment to the catheter assembly is equal to the maximum distance from the transition segment to the catheter assembly; or the main segment comprises a wavy shape and a maximum distance from the main segment to the catheter assembly is greater than or equal to the maximum distance from the transition segment to the catheter assembly.

9. The medical device according to claim 7, wherein when the supporting member is in the radially expanded configuration, the second section comprises a curved shape, and wherein a distance from the second section to the catheter assembly increases and then decreases from the proximal end to the distal end.

10. The medical device according to claim 2, wherein the catheter assembly is provided with a second fluid passage,
wherein the wrap element comprises an inner wrap member and an outer wrap member, wherein the inner wrap member is sleeved over a portion of the outer surface of the supporting member, wherein the outer wrap member is sleeved over an exterior of the inner wrap member, wherein opposite axial ends of the outer wrap member are attached to axial ends of the inner wrap member, wherein an inflation lumen is formed between the outer and inner wrap members, and wherein the inflation lumen is in communication with the second fluid passage.

11. The medical device according to claim 10, wherein the second fluid passage comprises a first fluid sub-passage and a second fluid sub-passage, which are in communication with each other,
wherein: the catheter assembly further comprises a second tubular body and a third tubular body; the first tubular body is partially inserted through the second tubular body, each of the proximal and distal ends of the first tubular body is located outside the second tubular body, the first tubular body is able to axially move relative to the second tubular body; the first fluid sub-passage is formed between an outer surface of the first tubular body and an inner surface of the second tubular body; a first seal structure is disposed between the outer surface of the first tubular body and the inner surface of the distal end of the second tubular body; a lumen of the third tubular body forms the second fluid sub-passage; a proximal end of the third tubular body is attached to the second tubular body; and a distal end of the third tubular body is inserted within the inflation lumen and is attached to the wrap element.

12. The medical device according to claim 11, further comprising an engagement member attached to a proximal end of the sheath assembly, wherein the engagement member comprises a housing provided with a first lumen and a second lumen that are in communication with each other,
wherein a proximal end of the first tubular body is inserted through the first lumen and extends out of the housing, and the first tubular body is able to move along the first lumen, wherein a second seal structure is provided between the outer surface of the first tubular body and a luminal wall of a proximal end of the first lumen, wherein a proximal end of the second tubular body is attached to the luminal wall of the first lumen, and wherein the first fluid sub-passage is brought into communication with the second lumen.

13. The medical device according to claim 1, wherein an outer surface of the expandable assembly is provided with a drug coating layer.
